# EUROPEAN PATENT SPECIFICATION

(11) **EP 1 623 746 B1**
(45) Date of publication and mention of the grant of the patent: **14.04.2010**
(21) Application number: 04709790.2
(22) Date of filing: 10.02.2004
(51) Int. Cl.: B01D 3/14, C07C 209/86

(54) **RECTIFYING PLANT FOR PURIFYING TERTIARY AMINES**
REKTIFIZIERUNGSANLAGE ZUR REINIGUNG VON TERTIÄREN AMINEN
INSTALLATION DE RECTIFICATION DESTINEE A L'EPURATION D'AMINES TERTIAIRES

(30) Priority: 11.03.2003 RU 2003106449
(43) Date of publication of application: 08.02.2006
(73) Proprietor: Nedd Marketing SA, Moscow, 129226 (RU)
(72) Inventor: MANKOVICH, Leonid, 26337 Kiryat-Motskin (IL); ZOTOV, Vyacheslav Ivanovich, Moscow, 109377 (RU)
(74) Representative: Ziebig, Marlene
(86) International application number: PCT/RU2004/000045
(87) International publication number: WO 2004/080563

(56) References cited:
- RU-C1- 2 145 596
- RU-C1- 2 200 151
- US-A- 2 447 746
- US-A- 2 895 886
- US-A- 4 543 163
- US-B1- 6 359 177

## Description

### (i) Field of the Invention.

The invention relates to organic chemistry, as well as to heat-mass-exchange equipment, and is applicable for distillation and treatment of various mixtures containing tertiary amine fractions C₈-C₂₅, some highly volatile components and heavy impurities.

### (ii) Background of the Invention.

Tertiary amines are widely used for production of a variety of organic synthesis products: water-repellent agents for oil recovery, detergents, antiseptics and sanitizers, quaternary ammonium compounds, etc. In particular, manufacturing of medical preparations with detergent and disinfectant properties requires raw materials of high quality: treated tertiary amines, essentially fractions C₁₂-C₁₄ with the base material content of at least 90%. In this case, it is commonly necessary to control the end product content of such fractions.

A mixture of commercial tertiary amines is multicomponent. It contains such impurities as heavy fractions of alkylamines, chloroalkanes, etc.

There is a known method of separation of cyclopentadiene dimer and methylcyclopentadiene dimer and an installation for implementation thereof [1], wherein a rectification column feed zone is connected with the top of a rotary film evaporator. Therein cyclopentadiene and methylcyclopentadiene monomers are separated from high-boiling hydrocarbons being essentially a heavy fraction which can be used as a heat carrier and a motor fuel constituent. The rectification column, in its turn, separates cyclopentadiene from methylcyclopentadiene. An overhead distillate is cyclopentadiene, and a bottom product is methylcyclopentadiene. Said technical solution employs the rotary film evaporator for separation of a light fraction, a mixture of cyclopentadiene and methylcyclopentadiene monomers, from a heavy fraction and for ducting the light fraction to the rectification column feed zone. Such technical solution is deficient in that it does not allow water and light fractions removal from the mixture of commercial tertiary amines, as well as separation of a narrow fraction, e.g., C₁₂-C₁₄, therefrom forasmuch as said mixture consists of more than ten different components. The task of separation of such mixture is further complicated by the fact that in some instances its components interact to form hard-to-cut azeotropes.

There is a known rectification installation [2] equipped with a stripping unit comprising a reboiler, a circulation line and a still bottoms discharge line connected to a counterflow film evaporator, the upper chamber of which is connected with a rectification column steam inlet. The film evaporator with forced still bottoms circulation under said technical solution is used to deplete still bottoms of a low-boiling component, and that allows to abate diffusion resistance to transfer of an easily boiling component of the still mixture and thereby to minimize concentration of the easily boiling components whose concentrations are on the point of the equilibrium value. This provides higher yield of useful product. However, such technical solution cannot be used for treating a mixture of tertiary amines, forasmuch as it only renders possible separation of light fractions together with the end product from heavy fractions, which remain in the still bottoms. Given that the end product in treating tertiary amines is the C₁₂-C₁₄ fraction, water and low-boiling fractions, particularly dimethylamine, must be separated before the target fraction is distilled, while still bottoms will contain a wide range of heavy fractions and high-temperature gumming products. Separation of the end product from light fractions, water and dimethylamine requires another rectification column, for it is on record that one rectification column allows cutting of a two-component mixture, and to cut a three-component mixture, one more column is required, and so on. [3]. It is obvious that for separation of a commercial mixture of tertiary amines, an installation must consist of ten rectification columns as a minimum. Such an installation would be unwieldy, complex and energy-intensive.

The closest prior art of the invention is a rectification installation [4] comprising a rectification column meant for receiving a base mixture containing tertiary amines, and for wet reflux. Said installation also includes a tank for the base mixture and a holding tank for refined amines. The above installation is deficient in that it is complex and ponderous, and forbids separation of mixtures containing tertiary amines with the number of carbon atoms in the alkyl radical exceeding 10.

A common disadvantage inherent to all above installations is that they forbid separation of a narrow fraction containing, for example, C₁₂-C₁₄, from the mixture of tertiary amines.

### (iii) Disclosure of the Invention.

It is an object of the invention to simplify the design, to improve efficiency of the installation, to provide for stability of the tertiary amines rectification process, to enhance treatment consisting in assurance of high purity of the end product, separation of a narrow fraction from the mixture, and flexibility of the end product composition.

This object is accomplished by that a rectification installation comprising a rectification column, means for feeding a separable mixture thereto and wet reflux, a tank for base mixture and a holding tank for the end product, as well as a still heating system, is complemented with a vacuum film evaporator, the upper chamber of which is connected with an outlet fitting of the base mixture tank and communicates with a vacuum system, and the lower chamber is connected with a means for feeding the base mixture to the column, the rectifying still therewith is furnished with a tube, the lower end of which is connected with the means for feeding the base mixture, and the upper end of the tube is arranged above the surface of the still fluid. Such a design makes it possible to pass the base mixture of tertiary amines through the film evaporator before feeding to the rectification column under vacuum, and then to deliver it to the surface of the still fluid with the aid of the metal tube. In the film evaporator, the base mixture is cleared from impurities of water and highly volatile components before it gets into the rectification column. Such impurities are removed from the upper chamber of the film evaporator. Its lower chamber receives a mixture containing the end product and heavy impurities. This reduces the likelihood of the column flooding, which is particularly important at a high rate of productivity. As the mixture flows along the metal tube, it undergoes a uniform preheating by the hot still fluid. Feeding of the base mixture free from highly volatile components through a layer of heavy still bottoms with a higher boiling temperature as compared with the boiling temperature of the incoming mixture makes it possible to increase its evaporation rate and to reduce power input for its heating to the boiling point. Arrangement of the tube above the surface of the still fluid enables spreading of the separable mixture on the still fluid surface in a uniform thin layer from which easily boiling products, including the target fraction, evaporate very quickly. The mixture coming to the rectification column is already fairly well-stripped of light and heavy impurities and enriched with the target fraction. In the rectification column, it undergoes the final treatment. As circumstances may require to adjust the composition of the end product and to provide the specified mixture ratio of the resultant narrow fraction of tertiary amines, the rectification product is subject to re-rectification until the specified mixture ratio is achieved. This increases the installation productivity and ensures its steady operation. The design is rather simple and compact.

### (iv) Description of the Drawing

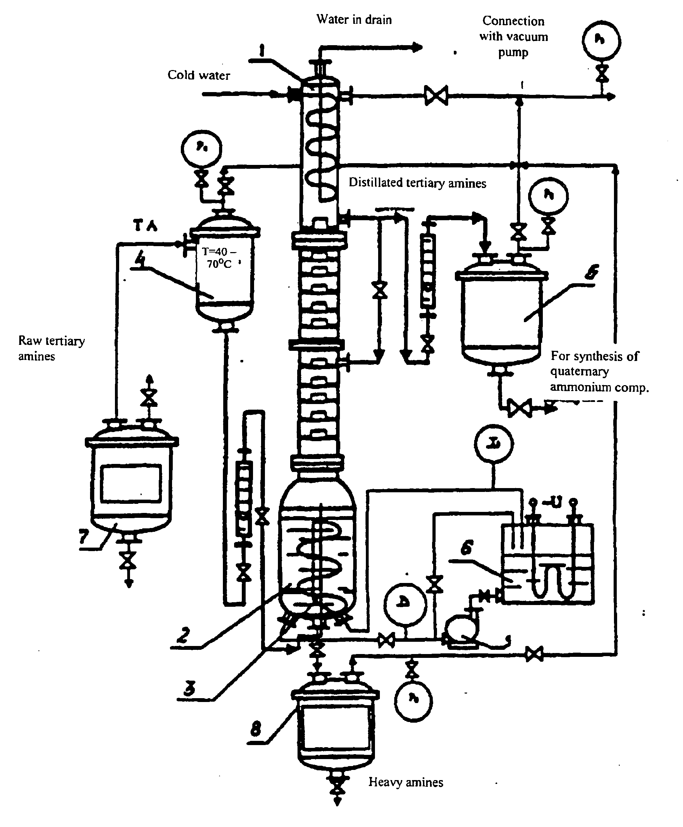

Fig. 1 is a diagram of an installation according to the invention.

An installation according to the invention consists of a rectification column 1 with a still 2, furnished with a metal tube 3, a film evaporator 4, a holding tank 5 for the end product, refined tertiary amines, a heater 6 with fire-bar elements filled with a thermal liquid T1, a tank 7 for a base mixture of commercial tertiary amines, a still residue downtank 8. The film evaporator 4, the rectification column 1 and the holding tank 5 for the end product communicate with a vacuum system.

### (v) Specific Embodiment

### An installation according to the invention operates as follows

A feedstock, a mixture of commercial tertiary amines, comes under vacuum from a tank 7 for a base mixture to a film evaporator 4, where a temperature of 40-70° C is maintained. In the film evaporator 4, water, dimethylamine and other easily boiling components of the base mixture boil already at 40° C due to a sudden drop in pressure. Vapors of the substances are evacuated from the upper chamber of the film evaporator 4 bypassing the effective volume of a column 1. The reduced mixture containing the target fraction of tertiary amines run down into the lower chamber of the film evaporator 4. Thereupon, said mixture comes through a metal tube 3 installed in a still 2 onto the surface of the still fluid. The metal tube 3 is installed so that its lower end is connected with a means for feeding the base mixture into the column 1, and the upper end thereof is arranged above the level of the still fluid. As the mixture goes up the tube, it warms up from the still fluid, which, in its turn, can be heated by a thermal liquid passing through a pipe coil or a jacket of the still 2. On the instant the mixture gets on the still fluid surface, it is already adequately warmed-up. Boiling of the mixture begins already inside the tube, and on the still fluid surface occurs fast evaporation of easily boiling components, including a narrow target fraction of tertiary amines, for example, C₁₂-C₁₄. Thus, the rectification column 1 receives the mixture as free as possible from light impurities removed in the film evaporator 4, as well as from heavy impurities remaining in the still 3, and containing essentially the end product, a narrow fraction of tertiary amines. The final treatment of the mixture occurs in the rectification column 1. The end product, a narrow fraction of treated tertiary amines with the base material content of at least 90 mass percent, accumulates on the top tray of the column 1. Thereon, part of the end product goes to a holding tank 5. The other part of the end product is sent to refluxing the column 1. The desired end product ratio of tertiary amines is provided by adjustment of the amount of reflux fed to the column 1, and the amount of the tapped end product, as well as by the number of rectification cycles. This allows to obtain a top quality product.

Furthermore, employment of the film evaporator that operates at a temperature well below the boiling point of the target fraction makes it possible to separate the flows of easily boiling impurities and the target fraction, thus reducing the vapor-phase load inside the column. This, in its turn, increases the column productivity as regards the target fractions of tertiary amines, and reduces the likelihood of carry-over and flooding of the column. The installation is simple, robust, compact, and cost-effective.

### List of References

1. Application RU No. 94023772, cl. C 07 C 13/15, 1996.
2. Patent RU No. 2182030, cl. B 01 D 3/00, 10.05.2002.
3. A.G. Kasatkin, Basic Processes and Apparatuses of Chemical Engineering, Moscow, 1961, p. 566.
4. Patent RU No. 2145596, cl. C 07 C 209/84, 2000. - equivalent to US-A-5 808 159.

## Claims

1. A rectification installation for treating tertiary amines comprising a rectification column (1), means for feeding a separable mixture thereto and wet reflux, a tank for base mixture (7), a holding tank (5) for the end product, as well as a still heating system (6), **characterized in that** said rectification installation is complemented with a vacuum film evaporator (4) the upper chamber of which communicates with the base mixture tank (7) and a vacuum system, and the lower chamber thereof is connected with a means for feeding the base mixture to the column, the column still (2) therewith is furnished with a metal tube (3) installed so that its lower end is connected with the means for feeding the base mixture, and the upper end thereof is arranged above the surface of the still fluid.

## Patentansprüche

1. Rektifizieranlage zur Behandlung von tertiären Aminen, umfassend eine Rektifikationskolonne (1), Einrichtungen für die Zuführung einer trennbaren Mischung dorthin und den Rückfluss, einen Tank für die Ausgangsmischung (7), einen Auffangbehälter (5) für das Endprodukt sowie ein Heizsystem (6) für den Destillationsapparat, **dadurch gekennzeichnet, dass** die Rektifizieranlage mit einem Vakuumfilmverdampfer (4) ergänzt ist, dessen obere Kammer mit dem Tank für die Ausgangsmischung (7) und einem Vakuumsystem kommuniziert, und dessen untere Kammer mit einer Einrichtung für die Zuführung der Ausgangsmischung zur Kolonne verbunden ist, der Kolonnendestillationsapparat (2) dabei mit einem Metallrohr (3) versehen ist, das so installiert ist, dass sein unteres Ende mit der Einrichtung für die Zuführung der Ausgangsmischung verbunden und sein oberes Ende über der Oberfläche der Flüssigkeit des Destillationsapparats angeordnet ist.

## Revendications

1. Installation de rectification destinée à traiter des amines tertiaires, comprenant une colonne de rectification (1), un dispositif pour y alimenter un mélange séparable et un reflux mouillé, un réservoir (7) pour le mélange de base, un réservoir de maintien (5) pour le produit final ainsi qu'un système de chauffage (6) du distillateur, **caractérisée en ce que** ladite installation de rectification est complétée par un évaporateur à film sous vide (4), dont la chambre supérieure communique avec le réservoir de mélange de base (7) et un système à vide, et la chambre inférieure de celui-ci est reliée à un dispositif destiné à alimenter le mélange de base vers la colonne, et en outre le distillateur de la colonne (2) est équipé d'une tuyauterie métallique (3) installée de telle sorte que son extrémité inférieure est reliée au dispositif d'alimentation du mélange de base et son extrémité supérieure est disposée au-dessus de la surface du fluide du distillateur.
